# EUROPEAN PATENT APPLICATION

(11) **EP 4 847 059 A1**
(43) Date of publication of application: **02.12.2026**
(21) Application number: 26180095.7
(22) Date of filing: 21.05.2026
(51) Int. Cl.: A61M 1/16, A61M 1/36

(54) **A DEVICE FOR FILTERING BLOOD, A DEVICE FOR SELECTIVE HEATING OF BLOOD AND A METHOD FOR CONTROLLING A SELECTIVE HEATING OF BLOOD**

(30) Priority: 28.05.2025 EP 25179484
(71) Applicant: Stichting IMEC Nederland, 5656 AE Eindhoven (NL); UMC Utrecht Holding B.V., 3584 CS Utrecht (NL)
(72) Inventor: WIERINGA, Fokko, 6661 RE Elst (NL); VOLLENBROEK, Jeroen, 7523 RE Enschede, (NL); GARCIA BRAS, Joao, 3526 JV Utrecht (NL)
(74) Representative: AWA Sweden AB

(57) **Abstract**

According to an aspect of the present inventive concept there is provided a device for filtering blood, the device comprising: a first channel configured for transporting blood; a second channel configured for transporting a dialysis fluid; a filter configured for being in fluid connection with the first channel and the second channel and further configured to allow exchange of constituents of the blood and constituents of the dialysis fluid between the first channel and the second channel; a heater configured for selectively heating the blood, wherein the heater is configured for controlling a temperature difference between the blood and the dialysis fluid at a location of the filter for providing a blood temperature that is higher than a dialysis fluid temperature.

## Description

### Technical field

The present description relates to a device for filtering blood, a device for selective heating of blood and a method for controlling a selective heating of blood.

### Background

Patients with Chronic Kidney Disease (CKD) that advance to the end stage of CKD (also called kidney failure) either need a transplant kidney or a form of dialysis to survive. Otherwise, they die from fluid overload and strongly elevated toxic bodily waste products that accumulate in the body, because the kidneys cannot remove them sufficiently anymore. As there is a persistent lack of suitable donor kidneys and, on top of that, not every patient is medically eligible for transplantation, the mainstay kidney replacement therapy is dialysis. Peritoneal dialysis (PD) uses the peritoneal membrane as dialysis filter, but this naturally present membrane ages quickly after PD start, so that the most widely applied form of dialysis is hemodialysis.

Today, hemodialysis systems apply an extracorporeal blood circuit with polymer hollow fiber-based dialysis filters. The hemodialysis system and the filter must mimic the bodies glomerular function in relation to filtering efficiency.

### Summary

An objective of the present description is to provide a device for filtering blood. Specifically, it is an object to provide a device for filtering strongly bound blood products out from the blood. It is a further objective to provide a device for selective heating of blood. Moreover, it is an objective to provide a method for controlling the selective heating of blood.

These and further objectives of the invention are at least partly met by the invention as defined in the independent claims. Preferred embodiments are set out in the dependent claims

According to a first aspect, there is provided a device for filtering blood, the device comprising:
a first channel configured for transporting blood;
a second channel configured for transporting a dialysis fluid;
a filter configured for being in fluid connection with the first channel and the second channel and further configured to allow exchange of constituents of the blood and constituents of the dialysis fluid between the first channel and the second channel;
a heater configured for selectively heating the blood, wherein the heater is configured for controlling a temperature difference between the blood and the dialysis fluid at a location of the filter for providing a blood temperature that is higher than a dialysis fluid temperature.

It is a realization that the selective heating of the blood, inducing the temperature difference between the blood and the dialysis fluid at a location of the filter, may ensure that a free fraction of otherwise strongly protein-bound toxins in the blood is high. These so-called protein-bound uremic toxins are by themselves (i.e. in an unbound, free state) small enough to pass the dialysis membrane, but because their high binding affinity to useful blood proteins (which should not pass the dialysis filter) there is only a limited free fraction of these molecules in the blood. The free fraction of toxins corresponds to a fraction of toxins not being bound to another substance, compared to a total amount of toxins in the blood.

The free fraction of toxins may be efficiently removed from the blood. For instance, toxins being bound to another substance, such as a protein, may be part of a relatively large molecule, which may not be able to pass through the filter. In addition, a pore size of the filter may preferably be small such that relatively large-size molecules, such as the essential protein albumin, which are desired to be maintained in the blood, are not transported through the filter towards the dialysis fluid.

Thus, it is a realization that the device provides an efficient filtering of the blood, thanks to the selective heating of the blood.

Moreover, it is a realization that selective heating of blood may increase the Brownian motion-driven diffusion of all molecules small enough to pass through the filter pores along the temperature gradient and thus out of the blood. Thus, it is a realization that thanks to the selective heating providing a blood temperature that is higher than the dialysis fluid temperature, the removal of blood toxins may be more effective when moving along the temperature gradient towards lower temperature at the dialysis fluid side.

In addition, the temperature gradient may be advantageous in ensuring that excess fluid in the blood is efficiently passed through the filter to the dialysis fluid.

The device may be configured for providing selective heating of the blood in close proximity to the filter, such as providing selective heating of the blood in the first channel. This implies that a high free fraction of toxins may be formed when the blood is close to the filter or at a location of the filter. Hence, the loosened toxins may not be able to become bound again before being filtered through the filter to the dialysis fluid.

The device may be a device for hemodialysis, hemofiltration or hemodiafiltration or a device for liver dialysis.

The blood may be blood from a human or an animal. The blood may be received immediately from blood vessels of the body. Thus, the first channel may be configured to be directly connected to a blood vessel, e.g., by the device being arranged implanted in the body, or by the device being connected to an extracorporeal blood circulation. The blood may alternatively also be extracted from the pertaining human or animal and even be temporarily stored before being later provided to the device. The device may thus also be configured to receive the blood from a sample acquired from the body.

The blood comprises several constituents, such as essential proteins, nutrients and toxins. In the body, the kidneys have the role of filtering the blood such that essential proteins, nutrients and other useful constituents comprised in the blood are kept in the blood while toxins and other waste products are removed from the blood. The toxins and waste products may come from the breakdown of food, cell metabolism or from other processes of the body.

One example of toxins may be Protein Bound Uremic Toxins (PBUTs). PBUTs are toxic by themselves, but they also can bind to blood plasma proteins, and in doing so can either alter their conformational structure, or block binding sites for other valuable endogenous or exogenous substances. For instance, the ability of blood albumin to bind drugs and other lipophilic organic acids is decreased in chronic renal failure by the accumulation of PBUTs such as hippuric acid, indoxyl sulphate and 3-carboxy-4-methyl-5-propyl-2-furanpropanoic acid (CMPF). Thus, PBUTs may exacerbate an already co-existing medical condition associated with kidney disease. PBUTs by themselves are relatively small molecules, but they are difficult to remove from the blood as they have a very high affinity for essential large proteins in the blood, such as albumin. Albumin is an important protein comprised in the blood. Removing albumin in the process of removing PBUTs may be deteriorating for the health of the subject, as the body only can produce a very limited amount of albumin per day. To limit removal of albumin, blood filters are designed such that albumin has difficulties of passing the filter. It is a realization, that to improve the removal of PBUTs, while preventing albumin loss, increasing the free fraction of PBUTs in the blood is a good strategy.

It is a realization that selectively increasing the temperature of the blood may increase the free fraction of the PBUTs in the blood and thus increase the proportion of PBUTs which may be removed from the blood without simultaneously removing albumin.

It is a further realization that an increased free fraction of PBUTs should only be present when the blood is in the device, and not when the blood is returned to the body because the toxicity of PBUTs is larger in free form than bound to the blood proteins.

The first channel is configured for transporting blood. Thus, the first channel may be configured such that it has a sterile inside environment, allowing for transport of blood with no or limited contact with the outside of the device.

The first channel may comprise nanoporous polymer hollow fibers, such as cellulose, acetate cellulose, polyacrylonitrile, polymethylmethacrylate, polysulfone, polycarbonate, polyethylene, and polyamide. The first channel may comprise of 8000-12000 hollow fibers. The fiber inner diameter may be ranging between 180-300µm, and the wall thickness of the fibers may be ranging between 2-30µm.

The second channel is configured for transporting dialysis fluid. The second channel may be configured such that it has a sterile inside environment, allowing for transport of dialysis fluid with no or limited contact with the outside of the device.

The second channel may be in fluid contact with a store unit for the dialysis fluid. Thus, the dialysis fluid may be stored outside of the device, in a sterile environment, before reaching the second channel. Likewise, the second channel may be in fluid connection to a residual unit, where the dialysis fluid may arrive after the second channel.

For hollow fiber-based filters, typically the blood is transported through the inside of the hollow fibers, which are bundled and suspended in the second channel, comprised of an outer housing, transporting the dialysis fluid. However, as an alternative, the first and second channel may be configured as so-called inside-out filters where the second channel is comprised of the fiber cores, transporting the dialysis fluid inside the fiber cores and the first channel is comprised of the outside of the fiber cores, transporting the blood such that the blood surrounds the fiber bundles.

The first and the second channel may extend in parallel to each other. In particular, the first and the second channel may extend in parallel at a location of the filter, with the filter being arranged between the first and the second channel.

The inlet of the first channel may be arranged close to the outlet of the second channel and vice versa. Thus, the flow of the blood along the filter may have opposite direction from the flow of the dialysate along the filter, thus using the so called counterflow principle. However, according to an alternative, the flow of the blood and the flow of the dialysate may run in the same direction, thus as an alternative the inlet of the first channel may be arranged neighboring the inlet of the second channel.

The dialysis fluid may be water-based. Alternatively, the dialysis fluid may be albumin-based. An albumin-based dialysis fluid may be advantageous when the device is configured for liver dialysis.

The dialysis fluid may be optically transparent.

The dialysis fluid may comprise constituents. The constituents may be ions.

The filter is configured for being in fluid connection with the first channel and the second channel. Thus, the filter may be arranged between the first channel and the second channel. As an example, the filter may extend between the first channel and the second channel.

The filter may be a nanoporous filter.

The filter is further configured to allow exchange of constituents of the blood and constituents of the dialysis fluid between the first channel and the second channel. Thus, constituents of the blood may move through the filter and into the dialysis fluid and vice versa, depending on concentration gradients, pressure gradients and other driving forces. Those constituents may be toxins such as PBUTs. In this way, toxins such as PBUTs can be removed, whereas electrolyte concentrations can be normalized. The dialysis fluid is free of toxins and may comprise other ionic and/or molecular constituents at the desired normal healthy concentration and pH-value. This may for instance be ions such as sodium ions, potassium ions, calcium ions and bicarbonate ions, which serve the purpose of keeping an equilibrium between the blood and the dialysis fluid in the concentration of the ions. Thus, the ions may move in both directions, depending on the partial pressure of the ions in the blood and dialysis fluid, respectively. This may ensure that the blood is not depleted in essential ions when passing through the device.

The filter may be formed by wafer-based manufacturing from e.g. silicon, or a silicon-based material.

The filter may comprise a porous polymer hollow fiber-based material. The filter may comprise the walls of a plurality of hollow fibers. Thus, the filter may comprise of a bundle of hollow fibers. The hollow fibers may have a porous wall. An advantage of having a filter comprised of a bundle of hollow fibers is that the hollow fibers may provide a large porous filter surface area.

The first channel may be comprised inside hollow fibers of the filter, while the second channel may be comprised outside of the hollow fibers of the filter. Alternatively, the second channel may be comprised inside hollow fibers comprised in the filter, while the first channel may be comprised outside of the hollow fibers comprised in the filter.

The filter may comprise pores, such as through-going pores. The pores may have a diameter of 4 - 24 nm.

The heater is configured for selectively heating the blood. Thus, the heater is configured to provide energy at least to the first channel such that the blood is selectively heated. According to an embodiment, the heater is configured such that the heating is not directly increasing the temperature of the dialysis fluid. However, it should be realized that the heater may not be perfectly isolated from the dialysis fluid. Hence, the heater being configured to selectively heat the blood may be configured to at least provide more heating energy to blood than to dialysis fluid such that the temperature of the blood is increased to a higher extent than the temperature of the dialysis fluid.

The heater may be configured to provide significantly more heating energy to blood than to dialysis fluid, such as 10 times more heating energy directly to blood than to dialysis fluid.

It should also be realized that the heating of blood may cause indirect heating of the dialysis fluid by heat being provided from the blood to the dialysis fluid.

The heating of the blood may increase the free fraction of constituents of the blood, in particular PBUTs. Thus, increasing the temperature of the blood may improve filtering of these constituents from the blood to the dialysis fluid.

The heater is further configured for controlling a temperature difference between the blood and the dialysis fluid at a location of the filter for providing a blood temperature that is higher than a dialysis fluid temperature.

Thus, the selective heating leads to a temperature difference between the blood and the dialysis fluid. The temperature difference may improve the diffusion of constituents from the blood through the filter and to the dialysis, as that diffusion may be driven at least in part by a temperature gradient. The temperature difference results in a difference in Brownian motion.

The heater may be arranged to surround a housing of the device in which the first and second channels are arranged. However, the heater may alternatively be arranged in direct contact with the first channel.

A resistive heater, or an array of resistive heater, may be placed in the first channel. A heater and/or cooler component, such as a Peltier element, or an array of Peltier elements, may be in direct contact with the first channel and an indirect contact with the dialysis fluid in the second channel.

It is a realization that precisely controlled selective heating of the blood may allow for increased filtration of constituents in the blood without the breakage of sterility, without addition of external molecules, and without any other, e.g. overheating-induced, chemical alteration of the blood.

The device may further comprise a flow sensor configured for measuring the speed of flow of blood through the first channel. The flow sensor may further be configured for communicating with the heater, such that the heater ends the heating of the blood if the speed of the flow of blood is below a threshold speed. Moreover, the communication between the flow sensor and the heater may be configured for controlling the heater such that temperature difference between the blood and the dialysis fluid at the location of the filter is proportional to the speed of flow of the blood.

The heater may be configured for controlling the temperature difference between the blood and the dialysis fluid at a plurality of locations along the filter. This may allow for efficient control of the difference along an extension of the filter which in turn may allow for specific temperature differences to occur at different locations.

The device may further comprise a control unit. The control unit may be configured for controlling the temperature of the blood when it is inside the device and when it leaves the device, back to the body.

According to one embodiment the heater may comprise an optical radiation source configured for selectively heating the blood.

An advantage of this is that the selective heating of the blood may be made without any direct contact with the blood. Thus, the selective heating may be made without breaking sterile boundaries of the blood.

Thus, the selective heating of blood may be performed via optical power absorption. In other words, the selective heating of the blood may be based upon the differences between the spectral absorption of blood and dialysis fluid, which mainly may arise from the most dominant chromophore Hemoglobin and its' derivatives present in blood but not in the dialysis fluid.

The optical radiation source may comprise a monochromatic optical radiation source. The optical radiation source may be a LED light source, or a matrix thereof.

The optical radiation source may be arranged such that it irradiates the blood comprised in the first channel from several directions. Thus, the optical radiation source may be arranged at least around the first channel, such as around the device.

Thanks to using the optical radiation source for selective heating of the blood, the heating of the blood may be provided by a heater that is not necessarily arranged close to the first channel. The optical radiation source may be arranged surrounding a housing in which the first channel and the second channel are arranged.

It is a realization that optical heating of blood is a very fast process, allowing for a fast warm up and a fast cooldown. This allows the blood to quickly increase in temperature and thereby quickly allowing the constituents of the blood to be brought to a free fraction.

For example, for adult dialysis, the blood flow speed through a dialysis filter is typically 300ml/min and an adult dialyzer typically has 100ml internal blood volume. Thus, the blood will dwell about 20 seconds inside the filter. During those 20 seconds, the blood must have time to increase and decrease in temperature, to reach the sought effect of increasing the free fraction of the constituents of the blood while yet reducing the temperature back to the normal body temperature before reaching the body again.

According to one embodiment the optical radiation source may be configured for emitting optical radiation of a wavelength in a range of 365-1000 nm.

This may correspond to a frequency range between 300 and 857 THz.

The optical radiation wavelength may be chosen so that it has a limited absorption by water-based solutions, such as the dialysis fluid, and a much larger absorption by both hemoglobin and oxyhemoglobin. Moreover, the optical radiation wavelength may be chosen such that it may penetrate the filter, such as hollow fibers, to reach the blood which may be flowing through the first channel.

An advantage of this is that the blood may be selectively and directly heated, while the dialysis fluid remains at a cooler temperature which means that the temperature gradient between the relatively warm blood and the relatively cool dialysis fluid is kept, which may help drive the filtration of constituents from the blood into the dialysis fluid.

According to one embodiment the heater may be configured for selectively heating the blood such that the free fraction of toxins in the blood increases.

Thus, the constituents of the blood may be toxins.

Increasing the free fraction of toxins in the blood may allow easier exchange between the blood and the dialysis fluid. Moreover, increasing the free fraction of toxins in the blood means that toxins that are normally bound to the molecules in the blood may be filtered out without having to filter out the molecules they bind to as well.

This is in particular advantageous for toxins with a high binding affinity to blood proteins. If the toxins may be forced by the increased temperature to release the binding from the proteins, more toxins may be filtered out with less loss of proteins. As essential proteins, like albumin, are needed for the function of the blood, it is highly important to keep them in the blood.

A further advantage is that the selective heating of the blood simultaneously increases the Brownian motion driving force behind transport of all molecules that fit through the filter between the blood to the dialysis fluid.

According to one embodiment the filter may comprise optically translucent constituting elements.

Alternatively, a channel wall of the first channel is formed by an optically transparent material.

An advantage of this is that the filter may be arranged in several different ways, but still allowing the heater to selectively heat the blood. This is especially advantageous if the heater comprises an optical radiation source.

According to one embodiment the heater may comprise a contact heater configured for selectively heating the blood.

The contact heater may be arranged at an inlet of the first channel, such that the blood is heated immediately at the inlet of the first channel. Alternatively, or additionally, the contact heater may be arranged as a spatially distributed pattern of contact heaters inside the first channel. The spatially distributed pattern of contact heaters may be arranged on locations just upstream from the filter.

According to an embodiment, the contact heater may be a resistive heater.

Alternatively, according to an embodiment, the contact heater may comprise Peltier elements. The Peltier elements may be configured such that they selectively heat the blood side, but also selectively cool the dialysis fluid side.

According to one embodiment the filter may comprise a spatial pattern of a plurality of locally thinned areas where filter pores are formed.

This may be advantageous for forming filter pores of a size-selective range on the nanometer scale. This is especially suitable for preventing useful larger protein molecules from the blood to be filtered out of the blood.

According to one embodiment the heater may be configured for generating pulsatile heating.

It is a realization that the release of certain constituents of the blood, in particular PBUTs, shows a hysteresis effect. Thus, that the free fraction of constituents of the blood, such as PBUTs, increases with temperature but further stays elevated a bit longer than when the temperature has returned to the starting temperature. In other words, the release of the bound constituent into a free fraction in the blood is affected by the environment around the blood not only at each time point but at a time point earlier in time as well.

As an example, after an albumin solution is warmed up and starts cooling down, the free fraction of PBUTs for a short while remains elevated. In other words, the free fraction of PBUTs falls back slower than the temperature does.

An advantage of this is that any heat-induced damage to the blood is affected by how high the temperature was elevated, and for how long that elevation lasted. Using shorter pulsed heating thus may improve the free toxin fraction at a reduced risk for heat-induced damage.

According to one embodiment the device may comprise at least one temperature sensor configured for sensing at least one of: the temperature of the blood, or the temperature of the dialysate fluid or configured for sensing the temperature difference between the blood and the dialysis fluid.

The temperature sensor may be arranged inside the first channel. The temperature sensor may be arranged inside the second channel. The temperature sensor may be arranged to extend along the filter.

The temperature of the blood may be relevant to measure for several reasons, such as for establishing an increase between temperature of the blood at the inlet of the first channel and temperature of the blood when reaching the filter. However, further reasons may be to assure that the blood has returned to a lower temperature before reaching the outlet of the first channel such that it does not overheat the body when returning to the normal blood circulation of the body.

The temperature of the dialysis fluid may be relevant to sense as it is relevant for keeping the temperature gradient between the blood and the dialysis fluid.

According to one embodiment the device may further comprise a supply channel configured for introducing a solution of binding-competitor molecules into the first channel.

The supply channel may be arranged at an inlet of the first channel. The supply channel may be configured such that the sterility of the first channel is kept.

The solution of binding-competitor molecules may comprise bilirubin and/or curcumin.

An advantage of this is that the release of any constituents of the blood from being bound into a free fraction may be increased as the binding-competitor molecules may compete with the constituents of the blood and force them to release any binding to molecules in the blood.

According to one embodiment the device may further comprise an exit optical radiation source, configured for emitting optical radiation to induce photochemical breakdown of the binding-competitor molecules.

The exit optical radiation source may allow breaking down the binding-competitor molecules into harmless components before the blood leaves the device.

The device may further comprise an exit optical radiation source even if the device does not comprise a supply channel configured for introducing a solution of binding-competitor molecules into the first channel. The exit optical radiation source may allow for breaking down molecules already present in the blood. This may for instance be bilirubin already present in the blood.

The exit optical radiation source may have a wavelength chosen specifically for the binding-competitor used. For instance, the range may be 490-500nm for breaking down bilirubin or 400-500 nm for breaking down curcumin.

As an example, bilirubin is a molecule which is naturally formed within the body during the decomposition of heme. In elevated concentrations it is toxic, but at normal low concentrations it offers some protective effects against reactive oxygen species. Bilirubin is a competitor molecule to PBUTs regarding the binding sites on albumin. It can also be photochemically decomposed by green-blue light. Thus, adding bilirubin through the supply channel to increase the free fraction of PBUTs in the blood may increase the filtration of PBUTs from the blood. However, before leaving the device the bilirubin may be removed using light of a wavelength of 490-500 nm.

Further, this is advantageous if the subject is a liver disease patient or a newborn baby who is needing both to be treated for elevated levels of bilirubin in the blood and other blood filtering treatments, such a dialysis. Then this may be made in combination.

According to one embodiment the second channel may further be configured for cooling the blood after the blood has passed the heater.

The blood may be cooled back to the desired exiting blood temperature before the blood leaves the device to re-enter the blood circulation.

The second channel may be arranged such that the inlet of the second channel is arranged in parallel to the outlet of the first channel. This allows for a dialysis fluid of a cooler temperature to enter the second channel and through heat exchange with the blood in the first channel cool the temperature of the blood. Thus, the cooling of the blood may be made in a passive way, not requiring any additional energy supplied to the device.

It is an advantage that this may lower the risk for both overheating and undercooling the subject, as the blood may reach a pre-determined suitable temperature before leaving the device and entering the subject.

According to one embodiment the device is an implantable device for being implanted into the body of a human or an animal.

Thus, the device may be made in a small size such that it may be suitable for being implanted into the body of a human or an animal.

This is advantageous as the blood then does not have to leave the body. Having the blood leaving the body may come with difficulties in keeping sterility. Moreover, if the blood leaves the body through for instance a tubing and the tubing breaks or the blood return needle slips out, large volume of blood may be lost leading to a severe condition for the subject. Even further, having the device implanted into the body may allow for more freedom of the human or animal as it can be much easier and with much less risks be connected to an external dialysis fluid providing machine in comparison to the present state of the art which requires an extracorporeal blood circuit, because the filter is located outside of the body.

According to a second aspect, there is provided a device for selective heating of blood, the device comprising:
a heater arranged on a holder, the holder being configured for being used in combination with or mounted onto a dialysis apparatus, wherein the heater is configured for selective heating of blood passing through the dialysis apparatus, wherein the heater is configured to control a temperature difference between the blood and the dialysis fluid on at least one location of a dialysis filter for providing a blood temperature that is higher than a dialysis fluid temperature.

This aspect may generally present the same or corresponding advantages as the first aspect.

A certain advantage of this is that the heater may be used in combination with already present dialysis apparatuses. Then, the advantages of the heater may be combined with the normally used equipment, reducing the need for exchanging large devices.

The holder may be configured for being mounted in connection to a dialysis apparatus. The holder may for instance be mounted on an infusion pole, on a bed or chair or on the dialysis apparatus.

The heater allows for selective heating of blood, such that constituents of the blood may be released from being bound to the blood. This may increase the filtering potential of the device.

For instance, the free fraction of PBUTs in blood is increasing with an increased temperature. The free fraction may be easier to filter out than PBUTs being bound to blood proteins. Additionally, the filtering out of scarce and essential blood protein may be reduced as the PBUTs are no longer bound to those proteins.

Further, the selective heating of the blood allows for the dialysis fluid to be kept at a lower temperature. This is advantageous as that will create temperature gradient which further drives the filtering out of constituents, such as toxins, from the blood. Even further, this allows for the dialysis fluid to work as a cooling fluid, such that the blood may by heat exchange to the dialysis fluid reach a cooler temperature before reaching the subject.

According to a third aspect, there is provided a method for controlling a selective heating of blood, the method comprising:
receiving blood of a first temperature in a first channel;
receiving dialysis fluid of a second temperature in a second channel;
filtering constituents of the blood and constituents of the dialysis fluid through a filter, wherein the filter is arranged in fluid connection to the first channel and the second channel;
selectively heating the blood with a heater;
controlling a temperature difference between the blood and the dialysis fluid on at least one location of the filter for providing a blood temperature that is higher than a filtering fluid temperature.

This aspect may generally present the same or corresponding advantages as the first and second aspect.

The method allows for efficient filtering of constituents out from the blood, thanks to the control of the selective heating of the blood.

The blood may be received from a human or from an animal.

When the blood is received, before being selectively heated, it may be having a temperature of around 37 °C. However, it should be understood that the temperature may vary. For instance, different animals may have a variation in the body temperature. Also, if the first channel is arranged outside the body, the blood may have been cooled down by being transported in a channel in room temperature.

The dialysis fluid may have a lower temperature than the blood. Thus, the second temperature may be lower than the first temperature. However, the first and second temperature may be almost the same.

The filter may be arranged extending between the first and the second channel. However, the filter may alternatively be arranged as hollow fibers where the first channel is arranged inside of the hollow fibers and the second channel is arranged on the outside surrounding the hollow fibers. Alternatively, the second channel is arranged inside hollow fibers while the first channel is arranged surrounding the hollow fibers.

Alternatively, the filter may be manufactured from a wafer, with blood flowing on one side and dialysate fluid on the other side (a stack of such filters can also be applied).

Constituents are exchanged between the blood and the dialysis fluid, such that constituents of the blood being for instance toxic are filtered out from the blood.

The blood is selectively heated. Thus, the temperature of the blood increases from the first temperature to a higher temperature while the second temperature is not changed. This will give rise to a temperature difference between the blood and the dialysis filter at a location of the filter.

The temperature difference is controlled, such that the temperature difference between the blood and the dialysis fluid at a location of the filter is kept.

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Fig. 1a is a schematic view of a device for filtering blood.
Fig. 1b is a schematic view of a device for filtering blood.
Fig. 1c is a schematic view of a device for filtering blood.
Fig. 1d is a schematic view of a device for filtering blood.
Fig. 2 is a schematic view of a device for filtering blood.
Fig. 3 is a schematic view of an implantable device for filtering blood.
Fig. 4 is a schematic view of a device for selective heating of blood.
Fig. 5 is a schematic view of a method for filtering blood.

### Detailed description

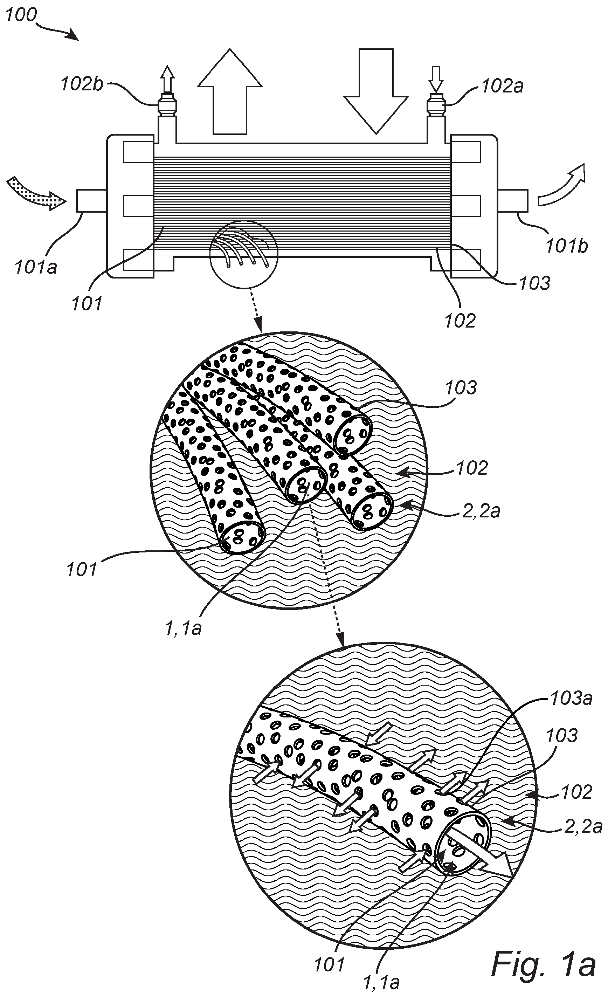

Fig. 1a to 1d illustrates a device 100 for filtering blood 1. The figures 1a to 1d will in the following be discussed together. The device 100 may be a device 100, such as a filter device 100, for hemodialysis or a device for liver dialysis. The blood 1 may be blood from a human or an animal.

The blood 1 may be received immediately from the body or from a sample acquired from the body. The blood 1 comprises several constituents 1a. The constituents of the blood 1a may be toxins such as Protein Bound Uremic Toxins (PBUTs). PBUTs are toxic small molecules by themselves, but they have a high binding-affinity to blood plasma proteins, and in doing so can either alter their conformational structure, or block binding sites for other valuable endogenous or exogenous substances.

The blood may have a temperature Tb of around 37 °C when received. However, it should be understood that the temperature Tb of the blood may be other temperatures than 37 °C.

The device 100 comprises a first channel 101 configured for transporting blood 1. Thus, the first channel 101 may be configured such that it has a sterile inside environment, allowing for transport of blood 1 with no or limited contact with the outside of the device 100.

The first channel 101 may comprise cellulose, acetate cellulose, polyacrylonitrile, polymethylmethacrylate, polysulfone, polycarbonate, polyethylene, and polyamide.

As illustrated in Fig. 1a, the first channel 101 is comprised inside a hollow fiber and the device 100 comprises several hollow fibers. Thus, the blood 1 is transported inside several hollow fibers arranged in parallel. The first channel may comprise of 8000-12000 hollow fibers. The fiber inner diameter may be ranging between 180-300µm, and the wall thickness of the fibers may be ranging between 2-30µm.

The first channel 101 has an inlet 101a and an outlet 101b. The blood 1 is received at the inlet 101a and leaves the first channel 101 through the outlet 101b.

The device 100 further comprises a second channel 102 configured for transporting a dialysis fluid 2. The second channel 102 may be configured such that it has a sterile inside environment, allowing for transport of dialysis fluid 2 with no or limited contact with the outside of the device 100.

As illustrated in Fig. 1a, the second channel 102 surrounds the first channel 101. However, it should be understood that the first channel 101 and the second channel 102 may be arranged in other ways in relation to each other. Alternatively, the device 100 may comprise a plurality of second channels 102 arranged to surround the first channel 101.

The second channel has an inlet 102a and an outlet 102b.

The second channel 102 may be in fluid contact with a store unit (not in picture) for the dialysis fluid 2. Thus, the dialysis fluid 2 may be stored outside of the device 100, in a sterile environment, before reaching the inlet 102a of the second channel 102. Likewise, the second channel 102 may be in fluid connection to a residual unit (not in picture), where the dialysis fluid 2 may arrive after exiting the second channel 102 through the outlet 102b. The second channel 102 may be arranged as a circuit, which may be arranged as a closed loop (not in picture), that removes toxins from the dialysis fluid exiting at 102b, to provide cleaned dialysis fluid back to re-enter at 102a.

As illustrated in Fig. 1a, the inlet 101a of the first channel 101 may be arranged close to the outlet 102b of the second channel 102. Thus, the flow of the blood 1 may have opposite direction from the flow of the dialysate 2. However, it may be arranged in a different way, such that for instance the inlet 101a of the first channel 101 and the inlet 102a of the second channel 102 may be arranged at close to each other. In such a case, the flow of the blood 1 and the flow of the dialysate 2 may run in the same direction.

The dialysis fluid 2 may be water-based. Alternatively, the dialysis fluid 2 may be albumin-based.

The dialysis fluid 2 may be transparent.

The dialysis fluid 2 may comprise constituents 2a such as ions.

The device 100 further comprises a filter 103 configured for being in fluid connection with the first channel 101 and the second channel 102. Thus, the filter 103 may be arranged between the first channel 101 and the second channel 102. As an example, the filter 103 may extend between the first channel 101 and the second channel 102.

The filter 103 may comprise a silicon-based material. The filter 103 may comprise a fiber-based material.

As is illustrated in Fig. 1a, the filter 103 may be formed in the walls of several hollow fibers with nanoporous walls.

The filter 103 may comprise an optically translucent constituting element.

The filter may comprise pores, such as through-and-through going pores 103a. The pores may have a diameter of 4 - 24 nm.

The filter 103 is further configured to allow exchange of the constituents of the blood 1a and constituents of the dialysis fluid 2a between the first channel 101 and the second channel 102. Thus, constituents of the blood 1a may move through the filter 103 and into the dialysis fluid 2. Those constituents 1a may be toxins such as PBUTs.

As is illustrated in Fig. 1a, the first channel 101 may be comprised inside hollow fibers of the filter 103, while the second channel 102 may be comprised outside of the hollow fibers of the filter 103. In this way, the second channel 102 may further be configured for cooling the blood 1 after the blood 1 has passed the heater 104. The blood 1 may be cooled back to the desired exiting blood temperature before the blood leaves the device 100 to re-enter the blood circulation in the body. The cooling may be made by setting the temperature Tf of the dialysis filter such that heat exchange can be made between the first channel 101 and the second channel 102. Thus, having the inlet 102a of the second channel 101 close to the outlet 101b of the first channel allows for control of the temperature Tf of the dialysis fluid 2 which is close to the blood 1 leaving the device.

Alternatively, the second channel 102 may be comprised inside hollow fibers comprised in the filter 103, while the first channel 101 may be comprised outside of the hollow fibers comprised min the filter 103.

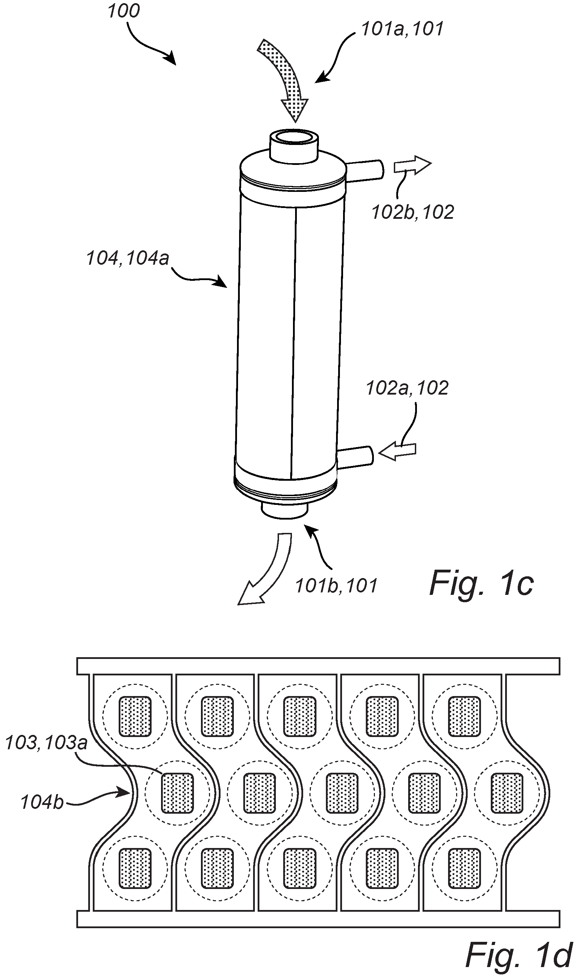

As an alternative, illustrated in Fig. 1d, the filter 103 may be formed as wafers 103 or diced sections of wafers, used as filter plates 103. Multiple filter plates 103 may be stacked on top of each other, with space in between for the first channel 101 to transport the flow of blood 1, and for the second channel 102 to transport dialysis fluid 2, respectively.. The filter plates 103 may comprise a spatial pattern of a plurality of locally thinned areas where filter pores 103a are formed.

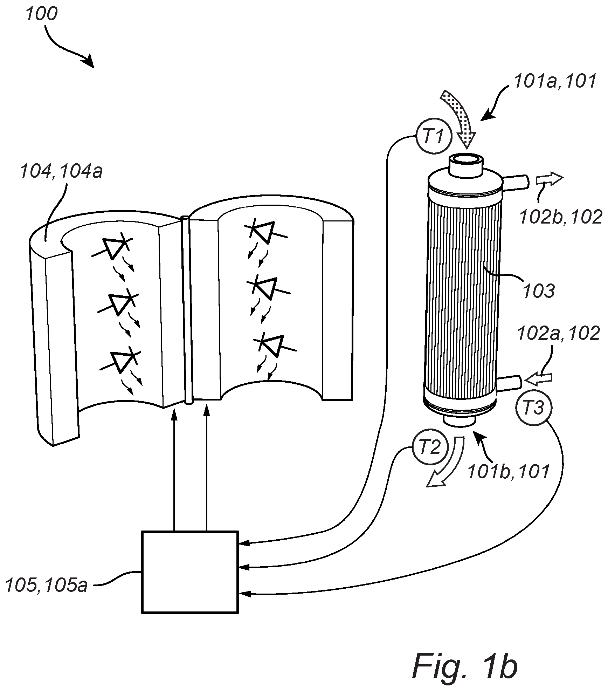

Illustrated in Fig. 1b is a simplified view of the device 100 from the outside and a heater 104 configured for selectively heating the blood 1. The heater 104 of Fig. 1b is configured for being arranged around a housing of the device 100 in which the first channel 101 and the second channel 102 are arranged. In Fig. 1c, the heater 104 is illustrated arranged around the housing of the device 100.

The heater 104 is configured to provide energy at least to the first channel 101 such that the blood 1 is selectively heated. In other words, the heater 104 is configured such that the heating is not directly increasing the temperature Tf of the dialysis fluid 2.

When the heater is arranged such as in Fig. 1b and 1c, the heater is configured such that it does not affect the temperature Tf of the dialysis fluid 2 even if the power from the heater 104 must be transported through the dialysis fluid 2 before reaching the blood 1.

The heater 104 is configured for controlling a temperature difference Td between the blood 1 and the dialysis fluid 2 at a location L of the filter 103 for providing a blood temperature Tb that is higher than a dialysis fluid temperature Tf.

The heater 104 of Fig. 1a and 1b comprise an optical radiation source 104a configured for selectively heating the blood 1. Thus, the selective heating of blood 1 may be performed via optical power absorption. In other words, the selective heating of the blood 1 may be based upon the differences between the spectral absorption of blood 1 and dialysis fluid 2, which mainly may arise from the most dominant chromophore hemoglobin and it's derivates, present in the blood 1 but not in the dialysis fluid 2.

The optical radiation source 104a may comprise a monochromatic light source. The optical radiation source 104a may be a LED light source or a plurality of LEDs. The optical radiation source 104a may be configured for emitting optical radiation of a wavelength in a range of 365-1000 nm.

As is illustrated in Fig. 1c, the optical radiation source 104a may be arranged such that it irradiates the blood from several directions. Thus, the optical radiation source 104a may be arranged at least around the first channel 101, such as around the housing of the device 100.

The heater 104 may be configured for selectively heating the blood 1 such that the free fraction of toxins in the blood 1 increases.

As an alternative, illustrated in Fig. 1d, the heater 104 may comprise a contact heater 104b configured for selectively heating the blood 1. The contact heater 104b may be a resistive heater or a Peltier element. The contact heater 104b may be arranged at an inlet of the first channel 101, such that the blood 1 is heated immediately at the inlet of the first channel 101. Alternatively, or additionally, the contact heater 104b may be arranged as a spatially distributed pattern of contact heaters inside the first channel 101. The spatially distributed pattern of contact heaters 104b may be arranged on locations just upstream from respective regions of the filter 103.

The heater 104 may be configured for generating pulsatile heating.

The device 100 may comprise a control unit 105. The control unit 105 may be configured for controlling the temperature Tb of the blood 1. The control unit may in particular be configured to control the temperature Tb of the blood 1 when it exits the first channel 101 through the outlet 101b.

The device 100 may further comprise at least one temperature sensor 105a configured for sensing at least one of: the temperature of the blood 1, or the temperature of the dialysis fluid 2 or configured for sensing the temperature difference between the blood 1 and the dialysis fluid 2.

The temperature sensor 105a may be arranged inside the first channel 101. The temperature sensor 105a may be arranged inside the second channel 102. The temperature sensor 105a may be arranged to extend along the filter 103.

The temperature sensor 105a may be connected to the control unit 105 such that the control of the heating of the blood 1 and the control of the temperature Tf of the dialysis fluid 2 may be interconnected.

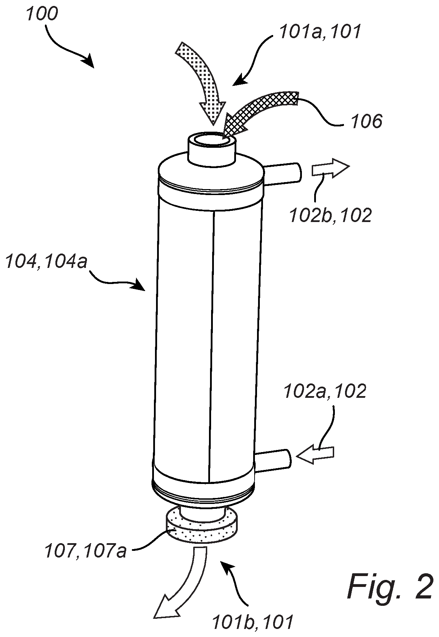

Fig. 2 illustrates a device 100 for filtering blood 2. The device 100 illustrated in Fig. 2 has many similarities with the device 100 illustrated in Fig. 1 and for simplicity, only the differences will be discussed below.

As illustrated in Fig 2, the device 100 further comprises a supply channel 106 configured for introducing a solution of binding-competitor molecules 3 into the first channel 101.

The supply channel 106 may be arranged at an inlet of the first channel 101. The supply channel 106 may be configured such that the sterility of the first channel 101 is kept.

The solution of binding-competitor molecules 3 may comprise bilirubin and/or curcumin.

The device 100 may further comprise an exit optical radiation source 107, configured for emitting optical radiation 107a to induce photochemical breakdown of binding-competitor molecules 3.

The exit optical radiation source 107 may allow to photochemically break down the binding-competitor molecules 3 into harmless components before the blood 1 leaves the device 100.

The exit optical radiation source 107 may have a wavelength chosen specifically for the binding-competitor 3 used. For instance, the range may be 490-500nm for breaking down bilirubin or 400-500 nm for breaking down curcumin.

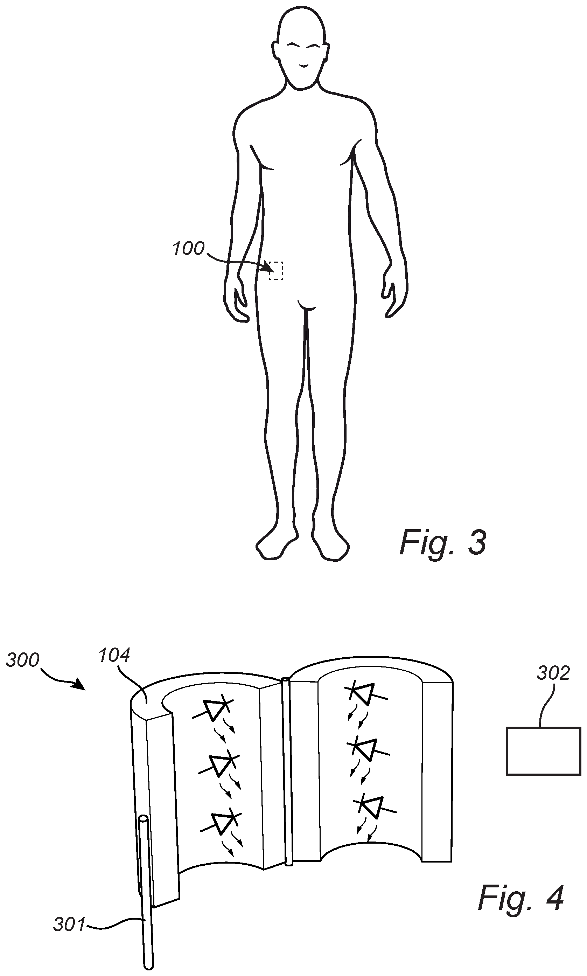

Fig. 3 illustrates an alternative embodiment where the device 100 is an implantable device 200 for being implanted into the body of a human or an animal.

Thus, the device 100 may be made in a small size such that it may be suitable for being implanted into the body of a human or an animal.

The first channel 101 may be arranged in fluid connection to the blood flow of the body, such that the blood may flow from the blood flow into the implantable device 200.

The second channel 102 may be arranged in fluid connection to a reservoir for carrying the dialysis fluid 2. The reservoir may be carried by the user.

Fig. 4 illustrates a device 300 for selective heating of blood 1.

The device 300 comprises a heater 104 arranged on a holder 301. The holder 301 is configured for being used in combination with or mounted onto a dialysis apparatus 302.

Thus, the holder 301 may be arranged on a dialysis apparatus 302. Alternatively, the holder 301 may be arranged in connection to the dialysis apparatus 302.

The heater 104 is configured for selective heating of blood 1 passing through the dialysis apparatus 302. Thus, the holder 301 may be arranged in such connection to the dialysis apparatus 302 that the heater 104 has power enough to heat the blood 1 in the dialysis apparatus 302.

The heater 104 is configured to control a temperature difference between the blood 1 and the dialysis fluid 2 on at least one location of a dialysis filter 103 for providing a blood temperature that is higher than a dialysis fluid temperature.

The device 300 may further comprise a temperature sensor 105 configured for sensing at least one of: the temperature of the blood 1, or the temperature of the dialysis fluid 2 or configured for sensing the temperature difference between the blood 1 and the dialysis fluid 2. The temperature sensor 105 may be arranged in relation to an inlet and/or output from the dialysis apparatus 302.

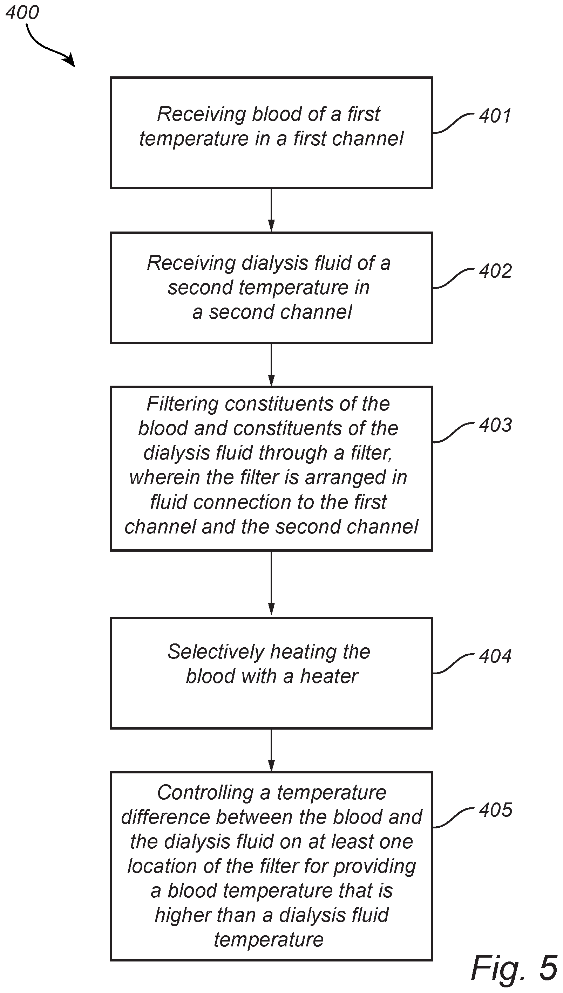

Fig. 5 illustrates a method 400 for controlling a selective heating of blood 1. The method 400 comprising receiving 401 blood 1 of a first temperature T1 in a first channel 101. The blood 1 may be received from a human or from an animal.

The first temperature T1 may be around 37 °C. However, it should be understood that the temperature T1 may vary. For instance, different animals may have a variation in the body temperature.

The first channel 101 may have a sterile inside environment, allowing for transport of blood 1 with no or limited contact with the outside of the device 100.

The first channel 101 may comprise cellulose, acetate cellulose, polyacrylonitrile, polymethylmethacrylate, polysulfone, polycarbonate, polyethylene, and polyamide.. The first channel 101 may comprise of 8000-12000 hollow fibers. The fiber inner diameter may be ranging between 180-300µm, and the wall thickness of the fibers may be ranging between 2-30µm.

The method 400 further comprises receiving 402 dialysis fluid 2 of a second temperature T2 in a second channel 102.

The dialysis fluid 2 may be water-based. Alternatively, the dialysis fluid 2 may be albumin-based.

The dialysis fluid 2 may be transparent.

The dialysis fluid 2 may have a lower temperature T2 than the blood 1. Thus, the second temperature T2 may be lower than the first temperature T1. However, the first T1 and second temperature T2 may be almost the same.

The second channel 102 may have a sterile inside environment, allowing for transport of dialysis fluid 2 with no or limited contact with the outside of the device 100.

The second channel 102 may be in fluid contact with a store unit (not in picture) for the dialysis fluid 2. Thus, the dialysis fluid 2 may be stored outside of the device 100, in a sterile environment, before reaching the second channel 102. Likewise, the second channel 102 may be in fluid connection to a residual unit (not in picture), where the dialysis fluid 2 may arrive after the second channel 102.

The first 101 and the second channel 102 may extend in parallel to each other.

The inlet of the first channel 101 may be arranged close to the outlet of the second channel 102 and vice versa. Thus, the flow of the blood 1 may have opposite direction from the flow of the dialysate 2. However, the flow of the blood 1 and the flow of the dialysate 2 may run in the same direction, thus as an alternative the inlet of the first channel 101 may be arranged neighboring the inlet of the second channel 102.

The method 400 further comprises filtering 403 constituents of the blood 1a and constituents of the dialysis fluid 2a through a filter 103. The filter 103 is arranged in fluid connection to the first channel 101 and the second channel 102.

Thus, constituents of the blood 1a may move through the filter 103 and into the dialysis fluid 2. Those constituents 1a may be toxins such as PBUTs.

The filter 103 may comprise a silicon-based or other material suitable for wafer-based manufacturing. The filter 103 may comprise a fiber-based material. The filter 103 may comprise several hollow fibers. The filter 103 may be comprises an optically translucent constituting element.

The first channel 101 may be comprised inside hollow fibers of the filter 103, while the second channel 102 may be comprised outside of the hollow fibers of the filter 103. Alternatively, the second channel 102 may be comprised inside of hollow fibers comprised in the filter 103, while the first channel 101 may be comprised outside of the hollow fibers comprised min the filter 103.

The filter may comprise pores, such as through-and-through going pores. The pores may have a diameter of 4 - 24 nm.

The filter 103 may comprises a spatial pattern of a plurality of locally thinned areas where filter pores 103a are formed.

The method 400 further comprises selectively 404 heating the blood 1 with a heater 104.

The heater 104 provides energy at least to the first channel 101 such that the blood 1 is selectively heated. The heating may be made via contact heating. In other words, the heater 104 may be configured such that the heating is not directly increasing the temperature Tf of the dialysis fluid 2.

The method 400 further comprises controlling 405 a temperature difference Td between the blood 1 and the dialysis fluid 2 at a location L of the filter 103 for providing a blood temperature Tb that is higher than a dialysis fluid temperature Tf.

The temperature difference Td may be used to drive the filtering of constituents from the blood 1a into the dialysis fluid 2, as the Brownian motion of the constituents of the blood 1a is driven by the energy difference between the blood 1 and the dialysis fluid 2.

The dialysis fluid 2 may be used to cool the temperature of the blood Tb after passing the filter 103. Thus, the second temperature T2 may be chosen such that the energy transfer from the blood 1 to the dialysis fluid 2 decreases the temperature of the blood Tb before the blood 1 reaches a subject.

The controlling 405 of the temperature difference Td may be performed using a temperature sensor 105 for sensing at least one of: the temperature of the blood 1, or the temperature of the dialysis fluid 2 or for sensing the temperature difference between the blood 1 and the dialysis fluid 2.

The heater 104 may comprise an optical radiation source 104a for selectively heating the blood 1. Thus, the selective heating of blood 1 may be performed via contactless optical power absorption, without any breakage of sterility. In other words, the selective heating of the blood 1 may be based upon the differences between the spectral absorption of blood 1 and dialysis fluid 2, which mainly may arise from the most dominant chromophore hemoglobin and it's derivates, present in the blood 1 but not in the dialysis fluid 2.

The optical radiation source 104a may comprise a monochromatic light source. The optical radiation source 104a may be a LED light source. The optical radiation source 104a emit optical radiation of a wavelength in a range of 365-1000 nm. The optical radiation source 104a may be arranged such that it radiates the blood from several directions. Thus, the optical radiation source 104a may be arranged at least around the first channel 101, such as around the device 100.

The heater 104 may selectively heat the blood 1 such that the free fraction of toxins in the blood 1 increases.

As an alternative, the heater 104 may comprise a contact heater 104b for selectively heating the blood 1. The contact heater 104b may be a resistive heater or a Peltier element. The contact heater 104b may be arranged at an inlet of the first channel 101, such that the blood 1 is heated immediately at the inlet of the first channel 101. Alternatively, or additionally, the contact heater 104b may be arranged as a spatially distributed pattern of contact heaters inside the first channel 101. The spatially distributed pattern of contact heaters 104b may be arranged on locations just upstream from the filter 103.

The heater 104 may be configured for generating pulsatile heating.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A device (100) for filtering blood (1), the device (100) comprising:
a first channel (101) configured for transporting blood (1);
a second channel (102) configured for transporting a dialysis fluid (2);
a filter (103) configured for being in fluid connection with the first channel (101) and the second channel (102) and further configured to allow exchange of constituents of the blood (1a) and constituents of the dialysis fluid (2a) between the first channel (101) and the second channel (102);
a heater (104) configured for selectively heating the blood (1), wherein the heater (104) is configured for controlling a temperature difference (Td) between the blood (1) and the dialysis fluid (2) at a location (L) of the filter (103) for providing a blood temperature (Tb) that is higher than a dialysis fluid temperature (Tf).

2. The device (100) according to claim 1, wherein the heater (104) comprises an optical radiation source (104a) configured for selectively heating the blood (1).

3. The device (100) according to claim 2, wherein the optical radiation source (104a) is configured for emitting optical radiation of a wavelength in a range of 365-1000 nm.

4. The device (100) according to any one of the preceding claims, wherein the heater (104) is configured for selectively heating the blood (1) such that the free fraction of toxins in the blood (1) increases.

5. The device (100) according to any one of the preceding claims, wherein the filter (103) comprises optically translucent constituting elements.

6. The device (100) according to claim 1 or 4, wherein the heater (104) comprises a contact heater (104b) configured for selectively heating the blood (1).

7. The device (100) according to any one of the preceding claims, wherein the filter (103) comprises a spatial pattern of a plurality of locally thinned areas where filter pores (103a) are formed.

8. The device (100) according to any one of the preceding claims, wherein the heater (104) is configured for generating pulsatile heating.

9. The device (100) according to any one of the preceding claims, wherein the device (100) comprises at least one temperature sensor (105) configured for sensing at least one of: the temperature of the blood (1), or the temperature of the dialysis fluid (2) or configured for sensing the temperature difference between the blood (1) and the dialysis fluid (2).

10. The device (100) according to any one of the preceding claims, wherein the device (100) further comprises a supply channel (106) configured for introducing a solution of binding-competitor molecules (3) into the first channel (101).

11. The device (100) according to claim 10, wherein the device (100) further comprises an exit optical radiation source (107), configured for emitting optical radiation (107a) to induce photochemical breakdown of the binding-competitor molecules (3).

12. The device (100) according to any one of the preceding claims, wherein the second channel (102) is further configured for cooling the blood (1) after the blood (1) has passed the heater (104).

13. The device (100) according to any one of the preceding claims wherein the device (100) is an implantable device (200) for being implanted into the body of a human or an animal.

14. A device (300) for selective heating of blood (1), the device (300) comprising:
a heater (104) arranged on a holder (301), the holder (301) being configured for being used in combination with or mounted onto a dialysis apparatus (302), wherein the heater (104) is configured for selective heating of blood (1) passing through the dialysis apparatus (302), wherein the heater (104) is configured to control a temperature difference between the blood (1) and the dialysis fluid (2) on at least one location of a dialysis filter (103) for providing a blood temperature that is higher than a dialysis fluid temperature.

15. A method (400) for controlling a selective heating of blood (1), the method (400) comprising:
receiving (401) blood (1) of a first temperature (T1) in a first channel (101);
receiving (402) dialysis fluid (2) of a second temperature (T2) in a second channel (102);
filtering (403) constituents of the blood (1a) and constituents of the dialysis fluid (2a) through a filter (103), wherein the filter (103) is arranged in fluid connection to the first channel (101) and the second channel (102);
selectively (404) heating the blood (1) with a heater (104);
controlling (405) a temperature difference (Td) between the blood (1) and the dialysis fluid (2) at a location (L) of the filter (103) for providing a blood temperature (Tb) that is higher than a dialysis fluid temperature (Tf).
